(19) Europäisches Patentamt
European Patent Office
Office européen des brevets

(11) Publication number: 0 160 379 B1

(12) # EUROPEAN PATENT SPECIFICATION

(45) Date of publication of the patent specification: 29.06.88

(51) Int. Cl.⁴: C 07 C 69/653, C 07 C 67/14, C 07 C 121/75, C 07 C 120/00

(21) Application number: 85301932.1

(22) Date of filling: 20.03.85

(54) **2,3,5,6-tetrafluorophenyl (meth)acrylates and their production.**

(30) Priority: 27.03.84 JP 57354/84
29.03.84 JP 59348/84

(43) Date of publication of application:
06.11.85 Bulletin 85/45

(45) Publication of the grant of the patent:
29.06.88 Bulletin 88/26

(84) Designated Contracting States:
CH DE FR GB LI NL

(56) References cited:
DE-B-1 044 802

(73) Proprietor: Nippon Shokubai Kagaku Kogyo Co., Ltd, 1, 5-chome, Koraibashi Higashi- ku, Osaka-shi Osaka- fu 541 (JP)

(72) Inventor: Kaieda, Osamu, 406- 59, Todaiji Shimamoto- cho, Mishima- gun Osaka- fu (JP)
Inventor: Hirota, Koichi, c/o Kawazuraryo, 4-10 Nakanoshima- cho, Suita- shi Osaka- fu (JP)
Inventor: Okitaka, Isao Hanada Shataku 11- 40, 65, 4-chome Higashi- Asakayama- machi, Sakai- shi Osaka- fu (JP)
Inventor: Nakamura, Toshiaki, 5-15, 3-chome, Fukaeminami Higashinari- ku, Osaka- shi Osaka-fu (JP)

(74) Representative: SERJEANTS, 25 The Crescent King Street, Leicester, LE1 6RX (GB)

EP 0 160 379 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1988

## Description

### Technical Field

The invention relates to 2,3,5,6-tetrafluorophenyl acrylates, methacrylates and a method for their production.

### Background Art

Methods of synthesizing unsaturated organic acid esters from phenols which are not fluorine substituted and unsaturated organic acid chlorides are disclosed by Magagnini et al, Gazz Chim. Ital., 96, 1035 (1966); Sumrell et al, J. Amer. Chem. Soc., 81, 4310 (1959); and Banks, J. Org. Chem., 42, 3965 (1977). No fluorine-substituted organic acid ester has been reported.
An analogous process is disclosed in DE-B-1 044 802.

### The Invention

The invention provides as new compounds 2,3,5,6-tetrafluorophenyl (meth)acrylates represented by the formula I below wherein R denotes a hydrogen atom or methyl group and X a hydrogen atom or cyano group.

These compounds are produced by a method which comprises esterifying a (meth)acrylic acid chloride with a 2,3,5,6-tetrafluorophenol represented by the formula II below wherein M denotes a hydrogen atom or an alkali metal atom and X has the same meaning as above with acrylic acid chloride or methacrylic acid chloride.

Suitable alkali metal salts embrace lithium salts, sodium salts and potassium salts, but sodium salts are preferred.

The amount of (meth)acrylic acid chloride per mol of 2,3,5,6-tetrafluorophenol is generally from 1.0 to 2.0 mols, preferably from 1.05 to 1.6 mols. The reaction is generally carried out in an organic solvent. Examples of suitable organic solvents include methanol, ethanol, isopropanol, butanols, benzene, toluene, xylenes, carbon tetrachloride, and perchloroethylene. The concentration of the 2,3,5,6-tetrafluorophenol in the solvent is generally from 2 to 30 % by weight, preferably from 3 to 20 % by weight. The reaction system preferably contains a hydrogen chloride trapping agent. Examples of suitable hydrogen chloride trapping agents include lithium hydroxide, sodium hydroxide, potassium hydroxide, magnesium carbonate, triethylamine, pyridine, and molecular sieves. The amount of hydrogen chloride trapping agent preferably exceeds the theoretrical enough to combine with all the hydrogen chloride produced.

The esterification temperature is preferably from -20° to +120°C, more preferably from -5° to +90°C. The reaction time is preferably from 0.5 to 100 h, more preferably from 1 to 70 h.

### Industrial Application

The 2,3,5,6-tetrafluorophenyl (meth)acrylates of the invention excel in polymerizability, and so are usable as raw materials for homopolymers or copolymers. They can be converted into various useful fluorine-containing (meth)acrylate polymers. These fluorine-containing (meth)acrylate polymers possess special functions different from (meth)acrylate polymers containing no fluorine, for example resistance to heat and light, and flame retardance. Thus they can be used in plastics, coating materials and adhesives. They are also useful as monomers for the production of electron beam resists, optical fibre sheaths and organic photosensitive binders.

### Best Mode

#### Example 1

A three-neck flask was charged with 250 g (1.295 mols) of pentafluorobenzonitrile, 171 g (3.05 mols) of potassium hydroxide and 500 ml of 2-methyl-2-propanol as a solvent. Under an atmosphere of nitrogen gas, the contents of the flask were stirred at 70°C for 20 h of reaction. The reaction mixture was evaporated to dryness to expel the solvent. The residue was dissolved in 250 ml of water. The resultant aqueous solution was adjusted to pH 2 by dropwise addition of 6N sulfuric acid, and added to 500 ml of diethyl ether for the extraction of the organic substance. This extraction was repeated twice. The ether layer was dried with magnesium sulfate and then evaporated to dryness to expel the ether. The residual crystals were recrystallized from benzene to obtain 4-cyano-2,3,5,6-tetrafluorophenol (yield 81.3 mol-% based on penta-fluorobenzonitrile).

m.p. 128° - 129°C
$^{19}F$ NMR (solvent: acetone -$d_6$, external standard substance: trifluoroacetic acid)
δ = 61.4 ppm (doublet-multiplet, 2F)
δ = 84.7 ppm (doublet-multiplet, 2F)

In 200 ml of carbon tetrachloride, 19.1 g (0.100 mol) of this 4-cyano-2,3,5,6-tetrafluorophenol and 13.0 g (0.125 mol) of methacrylic acid chloride were dissolved. To the resultant solution was added 20 g of a molecular sieve 3 Å in diameter. The resultant mixture was refluxed with agitation for 48 hours. The reaction mixture so produced was cooled and filtered to separate the molecular sieve. The filtrate was dried with magnesium sulfate and evaporated to dryness in a rotary evaporator. There was obtained 21.7 g of 4-cyano-2,3,5,6-tetrafluorophenyl methacrylate (yield 83.8 % based on 4-cyano-2,3,5,6-tetrafluorophenol, purity 96.5 %).

**Analyses**

4-Cyano-2,3,5,6-tetrafluorophenyl methacrylate re-crystallized from benzene
Melting point 87° - 88°C
Elementary analyses

| | C(%) | H(%) | F(%) | N(%) |
|---|---|---|---|---|
| Calculated | 50.97 | 1.93 | 29.34 | 5.41 |
| Found | 50.8 | 1.98 | 29.1 | 5.44 |

$^{1}H$ NMR (solvent: $CCl_4$, internal standard substance: TMS)
$CH_3$
δ = 2.1 ppm (singlet, 3H)
$CH_2$ δ = 5.9 ppm (singlet, 1H)
δ = 6.4 ppm (singlet, 1H)
$^{19}F$ NMR (solvent: acetone -$d_6$, external standard substance: trifluoroacetic acid)
δ = 58.8 ppm (doublet - doublet $^{J}AB$, A'B' ≈ 21 Hz,
$^{J}AB$, A'B ≈ 10 Hz, 2F)
δ = 75.3 ppm (doublet - doublet, $^{J}BA$, B'A' ≈ 21 Hz,
$^{J}BA'$ B'A ≈ 10 Hz, 2F)
Mass spectrum
EI m/e = 259 (m+)
Infrared absorption spectrum (KBr)
2250 $cm^{-1}$ ($^{\nu}C \equiv N$)
1760 $cm^{-1}$ ($^{\nu}C = 0$)
1650 $cm^{-1}$ ($^{\nu}C = C$)
1500 $cm^{-1}$ ($^{\nu}F$ - benzene ring C = C)

**Example 2**

In 50 ml of methanol having 0.94 g (0.0236 mol) of sodium hydroxide dissolved therein, 3.0 g (0.0157 mol) of 4-cyano-2,3,5,6-tetrafluorophenol prepared as in Example 1 was dissolved. The resultant solution was kept with ice at 0°C. To the cooled solution, 1.80 g (0.0173 mol) of methacrylic acid chloride was slowly added dropwise. After this addition, the resultant mixture was stirred at 0°C for 2 h. It was then mixed with 50 ml of pure water and adjusted to pH 10 by addition of 20 % aqueous sodium hydroxide solution. The resulting white crystals were filtered, washed with cold water, and then dried to obtain 1.35 g of 4-cyano-2,3,5,6-tetrafluorophenyl methacrylate (purity 98 % and yield 32 mol-% based on 4-cyano-2,3,5,6-tetrafluorophenol).

**Example 3**

In 30 ml of benzene, 3.0 g (0.0157 mol) of 4-cyano-2,3,5,6-tetrafluorophenol prepared as in Example 1 and 1.80 g (0.0173 mol) of methacrylic acid chloride was dissolved. 1.73 g (0.0173 mol) of calcium carbonate was added as a hydrogen chloride trapping agent, and the resulting mixture was stirred under reflux for 32 h. The resultant reaction mixture was cooled and filtered to separate the calcium carbonate. The filtrate was dried with magnesium sulfate and evaporated in a rotary evaporator. There was obtained 2.6 g of 4-cyano-2,3,5,6-tetrafluorophenyl methacrylate (yield 63.9 % and purity 95.4 %).

**Example 4**

In 200 ml of carbon tetrachloride, 19.1 g (0.100 mol) of 4-cyano-2,3,5,6-tetrafluorophenol prepared as in Example 1 and 11.3 g (0.125 mol) of acrylic acid chloride were dissolved. 20 g of molecular sieve 3 A in diameter was added, and the mixture was stirred under reflux for 38 h. The resulting reaction mixture was cooled and filtered to separate the molecular sieve. The filtrate was dried with magnesium sulfate and evaporated to dryness in a rotary evaporator. These was obtained 15.2 g of 4-cyano-2,3,5,6-tetrafluorophenyl acrylate (yield 62.0 % based on 4-cyano-2,3,5,6-tetrafluorophenol, purity 96.4 %).

**Anaylses**

Boiling point 62° C (2 mmHg)
Melting point 21° - 23° C
Elementary analyses

| | C(%) | H(%) | F(%) | N(%) |
|---|---|---|---|---|
| Calculated | 48.98 | 1.22 | 31.02 | 5.71 |
| Found | 48.9 | 1.25 | 30.8 | 5.77 |

$^1H$ NMR (solvent: $CCl_4$, internal standard substance: TMS)
$\delta = 5.9 - 7.0$ ppm (multiplet, 3H)
$^{19}F$ NMR (solvent: acetone - $d_6$, external standard substance: trifluoroacetic acid)
$\delta = 58.7$ ppm (broad doublet, J-21Hz, 2F)
$\delta = 75.2$ ppm (broad doublet, J-21Hz, 2F)
Mass spectrum
EI m/e = 245 (M+)
Infrared absorption spectrum (neat)
2250 $cm^{-1}$ ($^{\nu}C \equiv N$)
1770 $cm^{-1}$ ($^{\nu}C = O$)
1630 $cm^{-1}$ ($^{\nu}C = C$)
1500 $cm^{-1}$ ($^{\nu}F$ - benzene ring C = C)

**Example 5**

In 30 ml of benzene, 3.82 g (0.020 mol) of 4-cyano-2,3,5,6-tetrafluorophenol prepared as in Example 1 and 2.26 g (0.025 mol) of calcium carbonate as a hydrogen chloride trapping agent were stirred under reflux for 40 h. The resultant reaction mixture was cooled and filtered to separate the calcium carbonate. The filtrate was dried with magnesium sulfate and then evaporated to dryness in a rotaly evaporator. There was obtained 2.8 g of 4-cyano-2,3,5,6-tetrafluorophenyl acrylate (yield 57.1 %, purity 95.8 %).

**Example 6**

In 200 ml of methanol having 4.32 g (0.108 mols) of sodium hydroxide dissolved therein, 12 g (0.0723 mol) of 2,3,5,6-tetrafluorophenol was dissolved. The resultant solution was kept at 0°c with ice. To the cooled solution, 8.32 g (0.0796 mol) of methacrylic acid chloride was slowly added dropwise. After completion of the addition, the mixture was stirred at 0° C for 1.5 h. The mixture was combined with 160 ml of pure water and adjusted to pH 10 by addition of 20 % aqueous sodium hydroxide solution. The reaction mixture separated into two layers. An oil layer was washed again with pure water, dried with anhydrous sodium sulfate, and rectified with a precision fractionator. 12.1 g of 2,3,5,6-tetrafluorophenyl methacrylate was recovered as a fraction boiling at 56° to 57° C/3.5 mmHg, (yield 71.5 mol-% based on 2,3,5,6-tetrafluorophenol).

**Analyses**

Boiling point 56° - 57° C (3.5 mmHg)
Elementary analyses

| | C(%) | H(%) | F(%) |
|---|---|---|---|
| Calculated | 51.28 | 2.59 | 32.45 |
| Found | 51.3 | 2.56 | 32.3 |

$^1H$ NMR (solvent: $CCl_4$, internal standard substance: TMS)
$\delta$ = 2.08 ppm (singlet, 3H)
$\delta$ = 5.80 ppm (singlet, 1H)
$\delta$ = 6.35 ppm (singlet, 1H)
$\delta$ = 6.95 ppm (quintet, $J_{HF}$ = 9 Hz, IH)
$^{19}F$ NMR (solvent: acetone - $d_6$, external standard substance: trifluoroacetic acid)
$\delta$ = 64.4 ppm (broad singlet, 2F)
$\delta$ = 78.6 ppm (broad singlet, 2F)
Mass spectrum
El m/e = 234 (M+)
Infrared absorption spectrum (neat)
1760 $cm^{-1}$ ($^{\nu}C = O$)
1640 $cm^{-1}$ ($^{\nu}C = C$)
1490, 1530 $cm^{-1}$ ($^{\nu}F$ - benzene ring C = C)

**Example 7**

In 200 ml of carbon tetrachloride, 16.6 (0.100 mol) of 2,3,5,6-tetrafluorophenol and 13.0 g (0.125 mol) of methacrylic acid chloride were dissolved, and 20 g of molecular sieve 3 Å in diameter was added. The resultant mixture was stirred under reflux for 63 h, cooled and filtered to separate the molecular sieve. The filtrate was dried with magnesium sulfate and rectified with a precision fractionator. 19.8 g of 2,3,5,6-tetrafluorophenyl methacrylate was recovered as a fraction boiling at 56° to 57°C (3.5 mmHg) (yield 84.6 %, purity 98.5 %).

**Example 8**

In 40 ml of benzene, 4.1 g (0.025 mol) of 2,3,5,6-tetrafluorophenol and 3.9 g (0.0375 mol) of methacrylic acid chloride were dissolved, 3.75 g (0.0375 mol) of calcium carbonate was added as a hydrogen chloride trapping agent. The mixture was stirred under reflux for 46 h, cooled and filtered to separate the calcium carbonate, and the filtrate was rectified with a precision fractionator. 2.9 g of 2,3,5,6-tetrafluorophenyl methacrylate was recovered as a fraction boiling at 56° to 57°C/3.5 mmHg.

**Example 9**

In 200 ml of carbon tetrachloride, 16.6 g (0.100 mol) of 2,3,5,6-tetrafluorophenol and 11.3 g (0.125 mol) of acrylic acid chloride were dissolved, and 20 g of molecular sieve 3 Å in diameter was added. The resultant mixture was stirred under reflux for 60 h, cooled and filtered to separate the molecular sieve. The filtrate was dried with magnesium sulfate and rectified with a precision fractionator. 14.3 g of 2,3,5,6 tetrafluorophenyl acrylate was recovered as a fraction boiling at 46° to 47°C (3.5 mmHg) (yield 65.0 %, purity 98.8 %).

**Analyses**

Boiling point 46° - 47°C- (3.5 mmHg)
Elementary analyses

| | C(%) | H(%) | F(%) |
|---|---|---|---|
| Calculated | 49.11 | 1.84 | 34.53 |
| Found | 49.1 | 1.86 | 34.4 |

$^1H$ NMR (solvent: $CCl_4$, internal standard substance: TMS)
$\delta$ = 5.8 - 6.7 ppm (multiplet, 3H)
$\delta$ = 6.95 ppm (quintet, $J_{HF}$ = 9 Hz, 1H)
$^{19}F$ NMR (solvent: acetone - $d_6$,- external standard substance: trifluoroacetic acid)
$\delta$ = 64.3 ppm (multiplet, 2F)
$\delta$ = 78.5 ppm (multiplet, 2F)
Mass spectrum
El m/e = 220 (H+)

Infrared absorption spectrum (neat)
1770 $cm^{-1}$ ($^{\nu}C = O$)
1640 $cm^{-1}$ ($^{\nu}C = C$)
1490, 1530 $cm^{-1}$ ($^{\nu}F$ - benzene ring C = C)

**Example 10**

In 200 ml of methanol having 4.32 g (0.108 mol) of sodium hydroxide dissolved therein, 12 g (0.0723 mol) of 2,3,5,6-tetrafluorophenol was dissolved. The solution was kept at 0°C with ice. To the cooled solution, 7.2 g (0.0796 mol) of acrylic acid chloride was gradually added dropwise. After completion of the addition, the mixture was stirred at 0°C for 1.5 h, combined with 160 ml of pure water and adjusted to pH 10 by addition of 20 % aqueous sodium hydroxide solution. The reaction mixture separated into two layers. An oil layer was washed again with pure water, dried with anhydrous sodium sulfate, and rectified with a precision fractionator. 9.8 g of 2,3,5,6-tetrafluorophenyl acrylate was recovered as a fraction boiling at 46° to 47°C/3.5 mmHg (yield 61.6 mol-% based on 2,3,5,6-tetrafluorophenol).

The physical properties mentioned herein, and those in which the products of the invention have shown excellence, were determined as follows;

1. Melting points were measured by a melting point measuring device (FP-800 thermosystem) manufactured by Mettler.

2. NMR data was obtained by a Nuclear Magnetic Resonance (Varian XL-300) manufacturred by Varian.

3. Average molecular weight by Gel Permeation Column was measured by a high speed liquid chromatography (HPLC).

Column: HSG-20S (manufactured by K.K. Shimazu Seisakusho)

Solvent: Tetrahydrofuran: velocity 1 ml/min.

The measurement was carried by previously preparing a measuring line using standard polystyrene (commercially available) having various molecular weights.

4. Glass transition temperature (Tg) was measured under the following conditions:

Device: DT-40 (thermal analizing device manufactured by K.K. Shimazu Seisakusho) Sampling room: DSC-40 (Differential scanning colorimeter manufacturred by K.K. Shimazu Seisakusho) Range: 5 millijoule/sec; Temperature elevation speed: 10°C/min.

5. Refraction index $n^{20}_{D}$ was measurred by ATAGO ABBE 2T (measuring device for refraction index manufactured by Atago Co., Ltd).

**Claims**

1. A 2,3,5,6-tetrafluorophenyl (meth)acrylate represented by formula I:

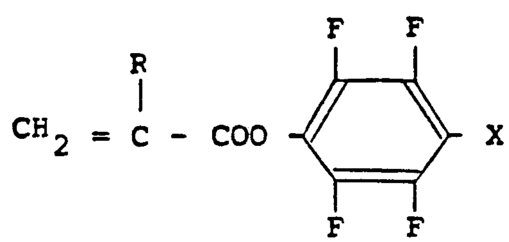

(I)

wherein R denotes a hydrogen atom or methyl group and X a hydrogen atom or cyano group.

2. A method for the production of a compound represented by the formula I above which comprises esterifying a tetrafluorophenol represented by formula II:

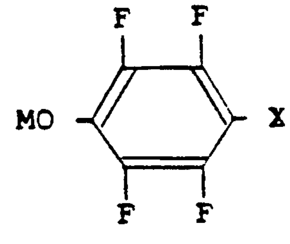

(II)

wherein M denotes a hydrogen atom or an alkali metal atom and X has the same meaning as in formula (I) above with acrylic acid chloride or methacrylic acid chloride.

3. A method according to claim 2 wherein the esterification is carried out in an organic solvent.

4. A method according to claim 2 to claim 3 wherein the esterification is carried out at from -5° to +90°C.

5. A method according to any of claims 2 to 4 wherein the esterification is carried out in the presence of a hydrogen chloride trapping agent.

**Patentansprüche**

1. Ein 2,3,5,6-Tetrafluorophenyl-(Meth)acrylat laut Formel I:

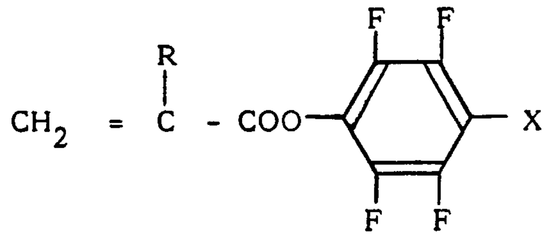

(I)

dadurch gekennzeichnet, daß R ein Wasserstoffatom bzw. eine Methylgruppe und X ein Wasserstoffatom bzw. eine Zyangruppe bedeuten.

2. Verfahren zur Herstellung einer Verbindung laut Formel I, das die Esterifizierung eines Tetrafluorophenols laut Formel II beinhaltet:

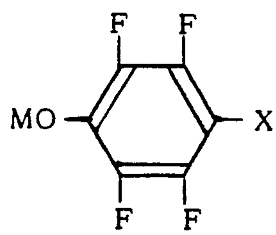

(II)

dadurch gekennzeichnet, daß M ein Wasserstoffatum bzw. Alkalimetallatom bezeichnet und X bei Acrylsäurechlorid oder Methacrylsäurechlorid das Gleiche wie in Formel (I) bedeutet.

3. Verfahren gemäß Anspruch 2, dadurch gekannzeichnet, daß die Esterifizierung in einem organischen Lösungsmittel erfolgt.

4. Verfahren gemäß Anspruch 2 bis 3, dadurch gekennzeichnet, daß die Esterifizierung zwischen -5° und +90°C erfolgt.

5. Verfahren gemäß einem der Ansprüche 2 bis 4, dadurch gekennzeichnet, daß die Esterifizierung in Gegenwart eines Wasserstoffchlorid-Fangmittels erfolgt.

**Revendications**

1. Un (meth)acrylate de tétrafluorophényle 2,3,5,6 représenté par la formule I:

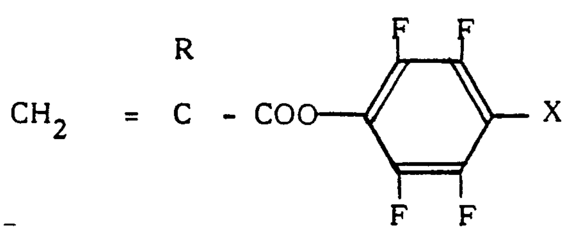

(I)

dans laquelle R indique un atome d'hydrogène ou groupe de méthyle et X un atome d'hydrogène ou groupe cyano.

2. Une méthode pour la production d'un composé représenté par la formule I ci-dessus qui comprend l'estérification d'un tétrafluorophénole représenté par la formule II:

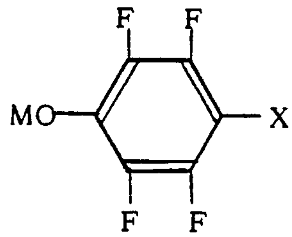

(II)

dans laquelle M indique un atome d'hydrogène ou un atome de métal alcalin et X à la même signification que dans la formule (I) ci-dessus avec du chlorure d'acide acrylique ou du chlorure d'acide méthacrylique.

3. Une méthode selon la revendication 2 dans laquelle l'estérification est effectuée dans un solvant organique.

4. Une méthode selon la revendication 2 et 3 dans laquelle l'estérification est effectuée de -5° à + 90°C.

5. Une méthode selon toutes les revendications 2 à 4 dans laquelle l'estérification est effectuée en présence d'un agent arrêtant le chlore d'hydrogène.